# EUROPEAN PATENT APPLICATION

(11) **EP 4 260 846 A1**
(43) Date of publication of application: **18.10.2023**
(21) Application number: 22168387.3
(22) Date of filing: 14.04.2022
(51) Int. Cl.: A61K 9/107, A61K 9/00, A61K 47/02, A61K 47/22

(54) **METAL-ORGANIC FRAMEWORKS AS SOLID SELF-MICROEMULSIFYING DRUG DELIVERY SYSTEMS**

(71) Applicant: Julius-Maximilians-Universität Würzburg, 97070 Würzburg (DE)
(72) Inventor: SCHELLER, Lena, 97070 Wuerzburg (DE); MEINEL, Lorenz, 97082 Wuerzburg (DE); LUEHMANN, Tessa, 97070 Wuerzburg (DE)
(74) Representative: Lederer & Keller Patentanwälte Partnerschaft mbB

(57) **Abstract**

The present invention relates to a composition comprising a metal-organic framework, one or more emulsifier(s) and one or more lipid(s). Furthermore, the present invention relates to a method for preparing a microemulsion in response to a physical- and/or chemical stimulus and to a method for increasing the solubility of a pharmaceutically active ingredient in a solvent and/or for improving the bioavailability of a pharmaceutically active ingredient and/or for preparing a self-microemulsifying drug delivery system. Finally, the present invention relates to the use of a composition comprising a metal-organic framework, one or more emulsifier(s) and one or more lipid(s) for preparing a microemulsion in response to a physical- and/or chemical stimulus and the use of the composition for increasing the solubility of a pharmaceutically active ingredient in a solvent and/or for improving the bioavailability of a pharmaceutically active ingredient and/or for preparing a self-microemulsifying drug delivery system.

## Description

### Field of the invention

The present invention relates to a composition comprising a metal-organic framework, one or more emulsifier(s) and one or more lipid(s). Furthermore, the present invention relates to a method for preparing a microemulsion in response to a physical- and/or chemical stimulus and to a method for increasing the solubility of a pharmaceutically active ingredient in a solvent and/or for improving the bioavailability of a pharmaceutically active ingredient and/or for preparing a self-microemulsifying drug delivery system. Finally, the present invention relates to the use of a composition comprising a metal-organic framework, one or more emulsifier(s) and one or more lipid(s) for preparing a microemulsion in response to a physical- and/or chemical stimulus and the use of a composition for increasing the solubility of a pharmaceutically active ingredient in a solvent and/or for improving the bioavailability of a pharmaceutically active ingredient and/or for preparing a self-microemulsifying drug delivery system.

### Technical Background

Highly potent but poorly water-soluble drug candidates are common and represent a major challenge for new drug development. Poor water solubility leads to poor oral bioavailability however, bioavailability has important clinical implications as both pharmacological and toxic effects are proportional to dose and bioavailability. Numerous physicochemical approaches to improve the oral bioavailability of such drugs have been explored such as particle size reduction, complexation, salt formation and addition of co-solvents. Since these methods have limited applicability for some compounds, formulation optimization approaches have been sought. Lipid-based formulations, such as microemulsions and self-microemulsifying drug delivery systems (SMEDDS) have already been extensively investigated (see non-patent literature 1 and 2).

Microemulsions are mixtures of lipids, one or more emulsifiers, optionally one or more co-emulsifier(s) and water. They form spontaneously, are thermodynamically stable and have a droplet size between 5 and 100 nm, which is why they appear clear to slightly opalescent to the naked eye. A distinction is made between oil-in-water (O/W) and water-in-oil (W/O) microemulsions, where oil or water, respectively, is present as fine droplets distributed in the other phase. This is made possible by the presence of emulsifiers. As a result, they are able to incorporate a wide variety of active ingredients and are used as drug carriers, especially for substances that are difficult to dissolve in water, whereby they are dissolved in an O/W microemulsion and their absorption and permeation can be improved (see non-patent literature 3).

Another form of lipid-based formulations are the so-called self-microemulsifying drug delivery systems (SMEDDS). These serve as preconcentrates for the formation of microemulsions, thus facilitating their delivery. These are isotropic mixtures of lipids, one or more emulsifiers and optionally one or more co-emulsifier(s) which, after oral administration with the aqueous gastrointestinal fluids, form in situ an O/W microemulsion with drug dissolved in the lipid droplets. Liquid SMEDDS can be administered using soft capsules with good compliance. Examples include Novartis' Sandimmun Neoral^{®} finished drug product for formulation of the immunosuppressant cyclosporine A and AbbVie's Norvir^{®} for delivery of the HIV protease inhibitor ritonavir (see non-patent literature 4).

The use of soft gelatin capsules has been established for many years, but manufacturers are confronted with stability and compatibility problems such as capsule leakage, seepage of ingredients into the shell and incompatibilities of the various components (see non-patent literature 5). These factors affect storage, shelf life and adequate release of the active ingredient and excipients, which is essential for the formation of a microemulsion (see non-patent literature 6).

These challenges have already been addressed in academia by converting the liquid SMEDDS into a solid dosage form. Here, methods such as adsorption to a carrier material, joint spray drying or 3D printing processes are used (see non-patent literature 7). For example, a SMEDDS containing cyclosporine A could be converted to a solid dosage form by simple absorption through porous magnesium aluminometasilicate (see non-patent literature 5). Similarly, naproxen as a liquid SMEDDS could be converted into smooth, granular particles by co-drying with maltodextrin, whose microemulsifying properties and dissolution profile were comparable to the liquid SMEDDS (see non-patent literature 8).

There is still a need for improved solide dosage forms for SMEDDS suitable for providing pharmaceutically active ingredients. Moreover, there is still a need for improving the bioavailability of poorly water-soluble pharmaceutically active ingredients.

### Non-patent literature

[1] S. Talegaonkar, A. Azeem, F.J. Ahmad, R.K. Khar, S.A. Pathan, Z.I. Khan, Microemulsions: a novel approach to enhanced drug delivery, Recent Pat Drug Deliv Formul, 2 (2008) 238-257.
[2] C.J .H. Porter, W.N. Charman, In vitro assessment of oral lipid based formulations, Advanced Drug Delivery Reviews, 50 (2001) S127-S147.
[3] M.J. Lawrence, G.D. Rees, Microemulsion-based media as novel drug delivery systems, Advanced Drug Delivery Reviews, 45 (2000) 89-121.
[4] S. Gibaud, D. Attivi, Microemulsions for oral administration and their therapeutic applications, Expert Opin Drug Deliv, 9 (2012) 937-951.
[5] C. Sander, P. Holm, Porous magnesium aluminometasilicate tablets as carrier of a cyclosporine self-emulsifying formulation, AAPS PharmSciTech, 10 (2009) 1388-1395.
[6] D.W. Kirn, J.H. Kang, D.H. Oh, C.S. Yong, H.G. Choi, Development of novel flurbiprofen-loaded solid self-microemulsifying drug delivery system using gelatin as solid carrier, J Microencapsul, 29 (2012) 323-330.
[7] K. Vithani, A. Goyanes, V. Jannin, A.W. Basit, S. Gaisford, B.J. Boyd, A Proof of Concept for 3D Printing of Solid Lipid-Based Formulations of Poorly Water-Soluble Drugs to Control Formulation Dispersion Kinetics, Pharm Res, 36 (2019) 102.
[8] K. Cerpnjak, A. Zvonar, F. Vrecer, M. Gasperlin, Development of a solid self-microemulsifying drug delivery system (SMEDDS) for solubility enhancement of naproxen, Drug Dev lnd Pharm, 41 (2015) 1548-1557.

### Summary of the invention

It is a problem of the present invention to provide a composition which can be used to prepare a microemulsions in response to a stimulus such as a physical and/or chemical stimulus. Specifically, it is a problem of the present invention to provide a composition which can be used as a solid dosage form of a self-microemulsifying drug delivery system, wherein a microemulsion is generated in response to a physical and/or chemical stimulus. Moreover, the composition according to the present invention can be used to provide a SMEDDS wherein any leakage and/or seepage of ingredients of the SMEDDS is avoided. Furthermore, the composition according to the present invention can be used to provide a solid dosage form of a SMEDDS which has excellent storage properties, shelf life and adequate release of the active ingredient(s) and excipient(s).

In addition, it is a problem of the present invention to provide a method for preparing a microemulsion in response to a stimulus such as a physical and/or chemical stimulus. Moreover, it is a problem of the present invention to provide a method for increasing the solubility of a pharmaceutically active ingredient in a solvent and/or for improving the bioavailability of a pharmaceutically active ingredient and/or for preparing a self-microemulsifying drug delivery system.

Finally, it is a problem of the present invention to provide the use of a composition for preparing a microemulsion in response to a physical- and/or chemical stimulus, for increasing the solubility of a pharmaceutically active ingredient in a solvent and/or for improving the bioavailability of a pharmaceutically active ingredient and/or for preparing a self-microemulsifying drug delivery system.

It has now been found that these and other problems can be solved by the composition and the methods of the present invention. The present invention relates to a composition comprising:
a) a metal-organic framework;
b) one or more emulsifier(s); and
c) one or more lipid(s).

Moreover, the present invention relates to a method for preparing a microemulsion in response to a physical- and/or chemical stimulus comprising the following steps:
i) providing a composition comprising:
   a) a metal-organic framework;
   b) one or more emulsifier(s);
   c) one or more lipid(s); and
   d) one or more solvent(s);
ii) applying a physical and/or chemical stimulus to the composition. Furthermore, the present invention provides the use of a composition comprising:
   a) a metal-organic framework;
   b) one or more emulsifier(s); and
   c) one or more lipid(s);
      for preparing a microemulsion in response to a physical- and/or chemical stimulus.

The present invention further relates to a method for increasing the solubility of a pharmaceutically active ingredient in a solvent and/or for improving the bioavailability of a pharmaceutically active ingredient and/or for preparing a self-microemulsifying drug delivery system comprising the following steps:
iii) providing a composition comprising:
   a) a metal-organic framework;
   b) one or more emulsifier(s);
   c) one or more lipid(s);
   d) one or more pharmaceutically active ingredient(s); and
   e) one or more solvent(s);
iv) applying a physical and/or chemical stimulus to the composition.

Finally, the present invention provides the use of a composition comprising:
a) a metal-organic framework;
b) one or more emulsifier(s); and
c) one or more lipid(s);
for increasing the solubility of a pharmaceutically active ingredient in a solvent and/or for improving the bioavailability of a pharmaceutically active ingredient and/or for preparing a self-microemulsifying drug delivery system.

The present invention is based on the recognition that in the composition of the present invention the one or more emulsifier(s), the one or more lipid(s), the one or more pharmaceutically active ingredient(s) and optionally the one or more co-emulsifier(s) are adsorbed to the metal-organic framework (MOF) and can be desorbed from the MOF in response to a physical and/or chemical stimulus (e.g. light, temperature, change in pH-value). Therefore, the composition of the present invention can be used to efficiently generate a microemulsion such as a water-in-oil or an oil-in-water microemulsion in response to a physical and/or chemical stimulus. Consequently, the composition according to the present invention can be used efficiently as a solid dosage form of a SMEDDS wherein the microemulsion is generated in response to a physical and/or chemical stimulus. The method for preparing a microemulsion in response to a physical- and/or chemical stimulus according to the present invention can efficiently be used to provide any microemulsion containing poorly soluble ingredients (such as pharmaceutically active ingredients). Finally, the present invention is based on the recognition that an efficient and reliable method for increasing the solubility of a pharmaceutically active ingredient in a solvent and/or for improving the bioavailability of a pharmaceutically active ingredient and/or for preparing a self-microemulsifying drug delivery system is provided, wherein a solid composition having excellent storage properties and shelf life is used which is capable of being triggered by a physical and/or chemical stimulus.

### Brief description of the drawings

Figure 1. XRPD diffractograms of unloaded and loaded ZIF-8 showing intact crystal structure of ZIF-8 after loading with drug and SMEDDS components. The diffractograms show the XRPD pattern of unloaded ZIF-8, ZIF-8 loaded with Nile Red and ZIF-8 loaded with SMEDDS MENIII, respectively.
Figure 2. Size of colloids before and after ZIF-8 synthesis. Circles show the z-average hydrodynamic diameter in nm of colloids (mean ± SD, n=3) after addition of SMEDDS MENIII to the 2-Methylimidazole solution. No colloidal structures in the supernatant were observed after synthesis and separation from the precipitate indicating the binding of the components of SMEDDS to ZIF-8.
Figure 3. Time dependent release of solubilized Nile Red in FaSSGF buffer pH 1.2 from MENIII@ZIF-8. Squares show the normalized concentration (mean ± SD, n=10) of solubilized Nile Red referred to the value before ZIF-8 synthesis as 100%.
Figure 4. Size of colloids in FaSSGF buffer pH 1.2 from MENIII@ZIF-8 over time. Circles show the z-average hydrodynamic diameter in nm of colloids (mean ± SD, n=10) indicating the formation of a microemulsion at pH 1.2.
Figure 5. XRPD diffractograms showing the structural changes of MENIII@ZIF-8 over time at pH 1.2.

### Detailed description of the invention

According to the present invention the term "about" means ±10% of the specified numeric value, preferably ±5% and most preferably ±2%.

According to the present invention the terms "SMEDDS", "liquid SMEDDS", "self-microemulsifying drug delivery systems" and "liquid self-microemulsifying drug delivery systems" have the meaning of preconcentrates for the formation of microemulsions, wherein these preconcentrates are isotropic mixtures of one or more lipids, one or more emulsifier(s) and optionally one or more co-emulsifier(s) and one or more pharmaceutically active ingredient(s) which, after oral administration with the aqueous gastrointestinal fluids, form in situ an O/W microemulsion with drug dissolved in the lipid droplets.

According to the present invention the terms "solid SMEDDS", "solid dosage form of a SMEDDS", "solid self-microemulsifying drug delivery system" and "solid dosage form of a self-microemulsifying drug delivery system" have the meaning of a solid composition wherein the one or more emulsifier(s), the one or more lipid(s) and optionally the one or more co-emulsifier(s) and the one or more pharmaceutically active ingredients are adsorbed to a solid compound such as a MOF.

The term "physical stimulus" means any physical stimulus such as electricity, temperature, pressure, light, sonic waves, x-rays, magnetic field, mechanical stress and so on.

The term "chemical stimulus" means any chemical stimulus such as changes in pH-value, initiating redox-processes, the presence of solvents, metals and enzymes and so on.

The term "co-emulsifier" means any compound capable of stabilizing a water-in-oil or oil-in-water microemulsion comprising an oil, water and one or more emulsifier(s).

The present invention relates to a composition comprising:
a) a metal-organic framework;
b) one or more emulsifier(s); and
c) one or more lipid(s).

The metal-organic framework may be any metal-organic framework known in the art. Preferably, the metal-organic framework is a pharmaceutically acceptable metal-organic framework. Preferably, the metal-organic framework is selected from the group consisting of: UiO-MOFs, ZIF-MOFs, MIL-MOFs, IRMOFs, HKUST-MOFs, COF-MOFs, BAF-MOFs, MFU-MOFs, ZJU-MOFs, TOF-MOFs, CAU-MOFs, MOF-Zr(DTBA) (the term "DTBA" means dithiobisbenzoic acid), MOF-5 and MOF-177. More preferably, the metal-organic framework is selected from the group consisting of: ZIF-MOFs, UiO-AZB (the term "AZB" means azobenzenedicarboxylate), ZJU-64, ZJU-800 and MOF-Zr(DTBA).

Still more preferably, the metal-organic framework is a zeolitic imidazolate framework (ZIF), which may be any ZIF known in the art. Most preferably, the metal-organic framework is ZIF-8.

Preferably, the metal-organic framework is present in the composition in an amount of from about 40wt% to about 99.90wt% based on the total weight of the composition, more preferably the MOF is present in the composition in an amount of from about 50wt% to about 99.50wt%, still more preferably of from about 55wt% to about 99.00wt%, even more preferably of from about 60wt% to about 95wt%, still even more preferably of from about 65wt% to about 90wt%, still even more preferably of from about 70wt% to about 85wt%, still even more preferably of from about 70wt% to about 80wt%, most preferably of from about 75wt% based on the total weight of the composition.

The one or more emulsifier(s) may be any emulsifier(s) known in the art. Preferably, the one or more emulsifier(s) are pharmaceutically acceptable emulsifier(s). Preferably, the one or more emulsifier(s) are non-ionic emulsifier(s), more preferably the one or more emulsifier(s) are pharmaceutically acceptable non-ionic emulsifier(s). Still more preferably, the one or more emulsifier(s) are selected from the group consisting of: Kolliphor^{®} RH40, Kolliphor^{®} EL, Capmul^{®} MCM, Captex^{®} 355, Labrafil^{®} M1944 CS; most preferably the one or more emulsifier(s) is Kolliphor^{®} RH40.

Preferably, the one or more emulsifier(s) are present in the composition in an amount of from about 0.01wt% to about 35wt% based on the total weight of the composition, more preferably of from about 0.02wt% to about 30wt%, still more preferably of from 0.05wt% to about 25wt%; even more preferably of from about 0.1wt% to about 20wt%, still even more preferably of from about 0.5wt% to about 17.5wt%, still even more preferably of from about 1.0wt% to about 17wt%; still even more preferably of from about 2wt to about 16.5wt%; still even more preferably of from about 2.5wt% to about 16wt%; most preferably of from about 5.0wt% to about 15wt% based on the total weight of the composition.

Preferably, the one or more emulsifier(s) are of from 1 to 5 emulsifier(s), more preferably of from 1 to 4 emulsifier(s), even more preferably of from 1 to 3 emulsifier(s), still more preferably 1 to 2 emulsifier(s), most preferably 1 emulsifier.

Preferably, the composition according to the present invention is a composition, wherein the one or more emulsifier(s) and the one or more lipid(s) are adsorbed to the metal-organic framework, and wherein the one or more emulsifier(s) and the one or more lipid(s) can be desorbed from the metal-organic framework in response to a physical and/or chemical stimulus. More preferably, the physical stimulus is selected from the group consisting of: temperature, pressure, light, sonic waves, x-rays, magnetic field and mechanical stress. More preferably, the chemical stimulus is selected from the group consisting of: a change in pH-value, initiating redox-processes and a presence of an enzyme.

The one or more lipid(s) may be any lipids known in the art. Preferably, the one or more lipid(s) are pharmaceutically acceptable lipids. Preferably, the one or more lipids are selected from the group consisting of: sesame oil, mono-, di- and triglycerids of C₁₀₋₂₆ fatty acids, PEG mono- and diesters of C₁₀₋₂₆ fatty acids.

Preferably, in the composition according to the present invention the one or more lipid(s) are present in amount of from about 0.01wt% to about 35wt% based on the total weight of the composition, more preferably of from about 0.02wt% to about 30wt%, still more preferably of from 0.05wt% to about 25wt%; even more preferably of from about 0.10wt% to about 20wt%, still even more preferably of from about 1.0wt% to about 17.5wt%, still even more preferably of from about 1.5wt% to about 17wt%; still even more preferably of from about 2.0wt to about 15wt%; still even more preferably of from about 2.5wt% to about 12.5wt%; most preferably of from about 5.0wt% to about 10wt% based on the total weight of the composition.

Preferably, the composition according to the present invention further comprises:
d) one or more pharmaceutically active ingredient(s).

The one or more pharmaceutically active ingredient(s) may be any pharmaceutically active ingredient(s) known in the art. Preferably, the pharmaceutically active ingredient(s) are poorly water-soluble. More preferably, the one or more pharmaceutically active ingredient(s) are selected from the group consisting of: Cyclosporin A, Ritonavir,Probucol, Vitamin K, Lapatinib and Fenofibrate.

Preferably, in the composition according to the present invention the one or more pharmaceutically active ingredient(s) are present in amount of from of from 1wt% to about 50wt% based on the total amount of the composition; more preferably of from about 2wt% to about 40wt%; still more preferably of from about 2.5wt% to about 35wt%; even more preferably of from about 4wt% to about 30wt%; still even more preferably of from about 5wt% to about 25wt%; still even more preferably of from about 5wt% to about 20wt%; most preferably of from about 5wt% to about 10wt% based on the total weight of the composition.

Preferably, the composition according to the present invention further comprises:
e) one or more co-emulsifier(s).

The one or more co-emulsifier(s) may be any co-emulsifier suitable for stabilizing a water-in-oil microemulsion or oil-in-water microemulsion known in the art. Preferably, the one or more co-emulsifier(s) are pharmaceutically acceptable co-emulsifier(s). More preferably, the one or more co-emulsifier(s) are selected from the group consisting of: polyethylene glycols, polypropylene glycols, polyethylene glcycol mono- and diethers, diethylene glycol mono- and diethers, C₂₋₁₀ alcohols and C₂₋₁₀ amines.

Preferably, the one or more co-emulsifier(s) are present in the composition in an amount of from about 0.001wt% to about 20wt% based on the total weight of the composition, more preferably of from about 0.002wt% to about 15wt%, still more preferably of from 0.005wt% to about 10wt%; even more preferably of from about 0.01wt% to about 10wt%, still even more preferably of from about 0.05wt% to about 7.5wt%, still even more preferably of from about 0.1wt% to about 5wt%; still even more preferably of from about 0.2wt to about 5wt%; still even more preferably of from about 0.5wt% to about 5wt%; most preferably of from about 1.0wt% to about 2.5wt% based on the total weight of the composition.

Preferably, the composition according to the present invention as defined herein is a pharmaceutical composition.

The composition according to the present invention may further comprise fillers or any pharmaceutically acceptable excipients known in the art.

Furthermore, the present invention relates to a method for preparing a microemulsion in response to a physical- and/or chemical stimulus comprising the following steps:
i) providing a composition comprising:
   a) a metal-organic framework;
   b) one or more emulsifier(s);
   c) one or more lipid(s); and
   d) one or more solvent(s);
ii) applying a physical and/or chemical stimulus to the composition.

In the method for preparing a microemulsion in response to a physical- and/or chemical stimulus in step i) in the provided composition the one or more emulsifier(s) and the one or more lipid(s) are adsorbed to the metal-organic framework and the one or more emulsifier(s) and the one or more lipid(s) can be desorbed from the metal-organic framework in response to a physical and/or chemical stimulus.

In the method for preparing a microemulsion in response to a physical- and/or chemical stimulus in step ii) a physical and/or chemical stimulus is applied to the composition of step i), wherein the physical and/or chemical stimulus leads to the desorption of the one or more emulsifier(s) and the one or more lipid(s) from the metal-organic framework. Upon the desorption of the one or more emulsifier(s) and the one or more lipid(s) a microemulsion is generated in the composition.

Generally, this method can be useful to generate microemulsions in response to a physical and/or chemical stimulus, for example to increase the solubility of poorly soluble ingredients additionally present in the composition.

The MOF in step i) may be any MOF as defined for the composition according to the present invention herein.

The one or more emulsifier(s) in step i) may be any emulsifier(s) as defined for the composition of the present invention herein.

The one or more lipid(s) in step i) may be any lipid(s) as defined for the composition of the present invention herein.

The one or more solvent(s) may be any solvent(s) immiscible with the one or more lipid(s). Preferably, in step i) the one or more solvent(s) are immiscible with the one or more lipid(s) and selected from the group consisting of: water, water containing solutions, and animal- or human body fluids such as gastrointestinal fluids.

Preferably, in step ii) the physical stimulus is selected from the group consisting of: temperature, pressure, light, sonic waves, x-rays, magnetic field and mechanical stress; more preferably the physical stimulus is selected from the group consisting of temperature, light and pressure. Preferably, in step ii) the chemical stimulus is selected from the group consisting of: a change in pH-value, initiating redox-processes and a presence of an enzyme; more preferably in step ii) the chemical stimulus is a change in pH-value or initiating a redox-process.

More preferably, in step ii) a chemical stimulus is applied to the composition, even more preferably in step ii) a chemical stimulus selected from the group consisting of: a change in pH-value, initiating redox-processes and a presence of an enzyme, is applied to the composition; more preferably the chemical stimulus is a change in pH-value or initiating a redox-process; most preferably, in step ii) a chemical stimulus is applied to the composition wherein the chemical stimulus is a change in pH-value.

Preferably, in the method for preparing a microemulsion in response to a physical- and/or chemical stimulus according to the present invention, in step i) the composition further comprises one or more co-emulsifier(s). The one or more co-emulsifier(s) may be any co-emulsifier(s) as defined for the composition according to the present invention herein.

Moreover, the present invention relates to the use of a composition comprising:
a) a metal-organic framework;
b) one or more emulsifier(s); and
c) one or more lipid(s);
for preparing a microemulsion in response to a physical- and/or chemical stimulus.

Preferably, the present invention relates to the use of a composition comprising:
a) a metal-organic framework;
b) one or more emulsifier(s); and
c) one or more lipid(s);
   in a method for preparing a microemulsion in response to a physical- and/or chemical stimulus comprising the following steps:
   i) providing a composition comprising:
      a) a metal-organic framework;
      b) one or more emulsifier(s);
      c) one or more lipid(s); and
      d) one or more solvent(s);
   ii) applying a physical and/or chemical stimulus to the composition.

Additionally, the present invention relates to a method for increasing the solubility of a pharmaceutically active ingredient in a solvent and/or for improving the bioavailability of a pharmaceutically active ingredient and/or for preparing a self-microemulsifying drug delivery system comprising the following steps:
iii) providing a composition comprising:
   a) a metal-organic framework;
   b) one or more emulsifier(s);
   c) one or more lipid(s);
   d) one or more pharmaceutically active ingredient(s); and
   e) one or more solvent(s);
iv) applying a physical and/or chemical stimulus to the composition.

In the method for increasing the solubility of a pharmaceutically active ingredient in a solvent and/or for improving the bioavailability of a pharmaceutically active ingredient and/or for preparing a self-microemulsifying drug delivery system in step iii) in the provided composition the one or more emulsifier(s), the one or more lipid(s), and the one or more pharmaceutically active ingredient(s) are adsorbed to the metal-organic framework and the one or more emulsifier(s), the one or more lipid(s), and the one or more pharmaceutically active ingredient(s) can be desorbed from the metal-organic framework in response to a physical and/or chemical stimulus.

In the method for increasing the solubility of a pharmaceutically active ingredient in a solvent and/or for improving the bioavailability of a pharmaceutically active ingredient and/or for preparing a self-microemulsifying drug delivery system in step iv) a physical and/or chemical stimulus is applied to the composition of step iii), wherein the physical and/or chemical stimulus leads to the desorption of the one or more emulsifier(s), the one or more lipid(s) and the one or more pharmaceutically active ingredient(s) from the metal-organic framework. Upon the desorption of the one or more emulsifier(s), the one or more lipid(s) and the one or more pharmaceutically active ingredient(s) a SMEDDS is generated which leads to the formation of a microemulsion in the composition, thereby increasing the solubility of a poorly soluble pharmaceutically active ingredient in a solvent and/or increasing the bioavailability of a pharmaceutically active ingredient.

The MOF in step iii) may be any MOF as defined for the composition according to the present invention herein.

The one or more emulsifier(s) in step iii) may be any emulsifier(s) as defined for the composition of the present invention herein.

The one or more lipid(s) in step iii) may be any lipid(s) as defined for the composition of the present invention herein.

The one or more pharmaceutically active ingredient(s) in step iii) may be any pharmaceutically active ingredients as defined for the composition according to the present invention herein.

The one or more solvent(s) may be any solvent(s) immiscible with the one or more lipid(s). Preferably, in step i) the one or more solvent(s) are immiscible with the one or more lipid(s) and selected from the group consisting of: water, water containing solutions, and animal- or human body fluids such as gastrointestinal fluids.

Preferably, in step iv) the physical stimulus is selected from the group consisting of: temperature, pressure, light, sonic waves, x-rays, magnetic field and mechanical stress; more preferably the physical stimulus is selected from the group consisting of: temperature, light and pressure. Preferably, in step iv) the chemical stimulus is selected from the group consisting of: a change in pH-value, initiating redox-processes and a presence of an enzyme; more preferably the chemical stimulus is a change in pH-value or initiating a redox-process; most preferably in step iv) the chemical stimulus is a change in pH-value.

More preferably, in step iv) a chemical stimulus is applied to the composition, even more preferably in step iv) a chemical stimulus selected from the group consisting of: a change in pH-value, initiating redox-processes and a presence of an enzyme, is applied to the composition; even more preferably in step iv) a chemical stimulus is applied to the composition wherein the chemical stimulus is a change in pH-value or initiating a redox-process; most preferably, in step iv) a chemical stimulus is applied to the composition wherein the chemical stimulus is a change in pH-value.

Preferably, in the method for increasing the solubility of a pharmaceutically active ingredient in a solvent and/or for improving the bioavailability of a pharmaceutically active ingredient and/or for preparing a self-microemulsifying drug delivery system according to the present invention, in step iii) the composition further comprises one or more co-emulsifier(s). The one or more co-emulsifier(s) may be any co-emulsifier(s) as defined for the composition according to the present invention herein.

Finally, the present invention relates to the use of a composition comprising:
a) a metal-organic framework; and
b) one or more emulsifier(s); for increasing the solubility of a pharmaceutically active ingredient in a solvent and/or for improving the bioavailability of a pharmaceutically active ingredient and/or for preparing a self-microemulsifying drug delivery system.

Preferably, the preset invention relates to the use of a composition comprising:
a) a metal-organic framework; and
b) one or more emulsifier(s);
   in a method for increasing the solubility of a pharmaceutically active ingredient in a solvent and/or for improving the bioavailability of a pharmaceutically active ingredient and/or for preparing a self-microemulsifying drug delivery system comprising the following steps:
   iii) providing a composition comprising:
      a) a metal-organic framework;
      b) one or more emulsifier(s);
      c) one or more pharmaceutically active ingredient(s); and
      d) one or more solvent(s);
   iv) applying a physical and/or chemical stimulus to the composition.

### Examples

### Example 1: Preparation of Preconcentrate MENIII (SMEDDS)

A liquid preconcentrate for a SMEDDS was prepared by mixing the following compounds:
- 40% (w/w) sesame oil:maisine (1:1)
- 50% (w/w) Kolliphor^{®} RH40
- 10% (w/w) PEG 400
- Fluorescent dye Nile Red

In this Example sesame oil:maisine represent the one or more lipids, Kolliphor^{®} RH40 represents the one or more emulisifier(s), PEG 400 represents the one or more co-emulsifier(s) and the fluorescent dye Nile Red represents an active ingredient, which in practice may be substituted e.g. by a pharmaceutically active ingredient.

### Example 2: MENIII@ZIF-8 preparation

A ZIF-8 MOF loaded with the liquid preconcentrate MENIII obtained from Example 1 was prepared according to the following procedure:
- 0.20 g SMEDDS MENIII was added to 33 mL water containing 1.80 g 2-Methylimidazole
- 0.58 g Zinc acetate dihydrate was dissolved in 17 mL water and added to the 2-Methylimidazole solution
- After gentle stirring the mixture was aged at 25 °C for 24h
- After 24h the precipitate was separated from the supernatant by centrifugation at 13.400 rpm for 20 min and dried at 60°C over night. The yield of the solid product was about 1 g. The composition was determined by weighing the solid product and inspecting the supernatant for loss of excipients after the separation from the precipitate.

### Precipitate and supernatant analysis

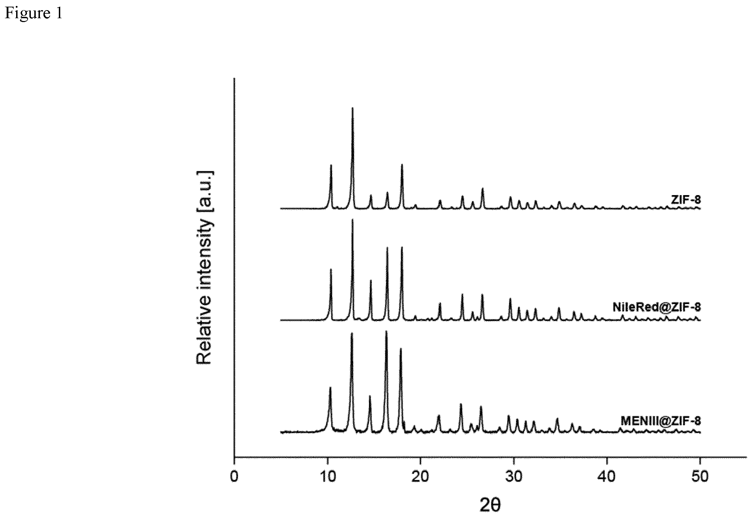

The precipitate obtained from Example 2 was analyzed via XRPD (Powder diffractometer) and the results are shown in Figure 1. XRPD diffractograms of unloaded and loaded ZIF-8 showing intact crystal structure of ZIF-8 after loading with drug and SMEDDS components. The diffractograms show the XRPD pattern of unloaded ZIF-8, ZIF-8 loaded with Nile Red and ZIF-8 loaded with SMEDDS MENIII, respectively. The results of the data shown in Figure 1 demonstrates that the metal-organic framework ZIF-8 was successfully synthesized in the presence of the SMEDDS MENIII.

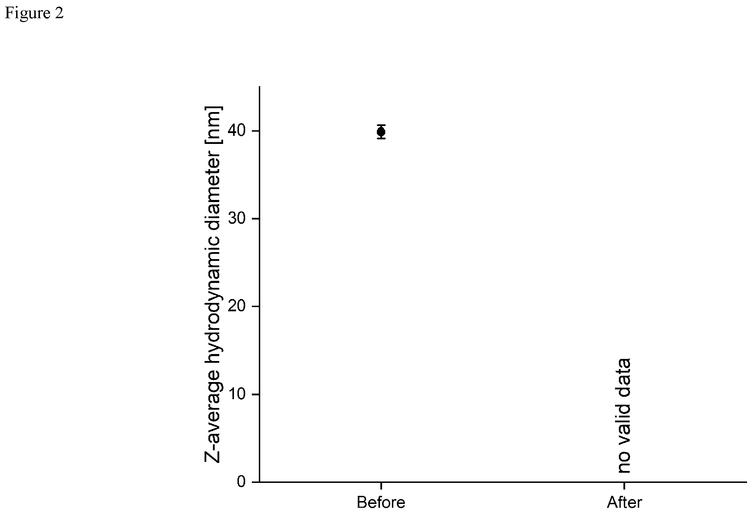

The supernatant obtained from Example 2 was analyzed via dynamic light scattering and the Z-average hydrodynamic diameter [nm] of colloids contained in the supernatant was measured. In Figure 2 the size of the colloids before and after ZIF-8 synthesis are shown. The circles show the Z-average hydrodynamic diameter [nm] of colloids of the emulsion containing the SMEDDS MENIII (mean ± SD, n=3) before the ZIF-8 synthesis. After the addition of SMEDDS MENIII to the 2-methylimidazole solution, the addition of Zinc acetate dihydrate to carry out the MOF synthesis and separation from the precipitate after 24h no colloidal structures were observed in the supernatant which demonstrates that the components of the SMEDDS MENIII were adsorbed to the MOF ZIF-8. Consequently, a MENIII loaded ZIF-8 was prepared wherein the MENIII ingredients were adsorbed to the MOF ZIF-8.

### Example 3: Microemulsion and drug release

The generation of a SMEDDS and the formation of a microemulsion containing the fluorescent dye Nile Red using the MENIII loaded ZIF-8 in response to a pH-value change which is a chemical stimulus was investigated.
- 50 mL FaSSGF buffer pH 1.2 was added to the obtained precipitate obtained from Example 2.
- The mixture was incubated at 37 °C and 700 rpm for 24h.
- Aliquots were taken at 0.10, 1, 2, 3, 4, 5, 6 and 24 h and analyzed by fluorescence intensity measurement, dynamic light scattering and XRPD.

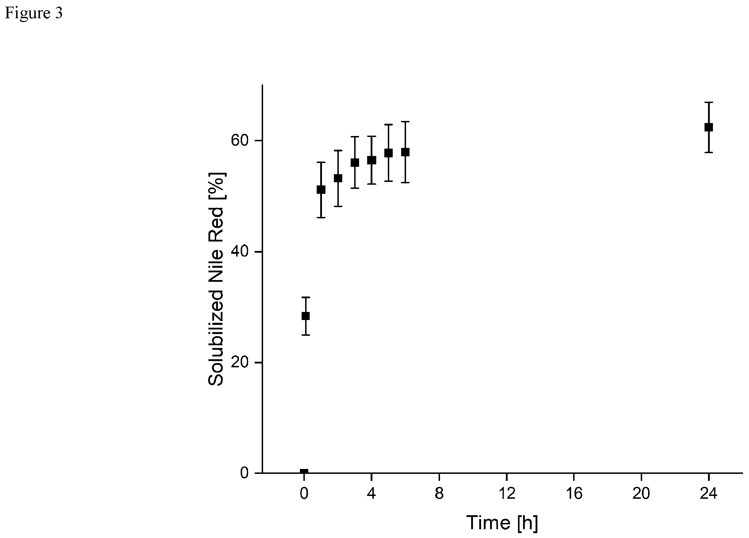

The results are shown Figures 3, 4 and 5. In Figure 3 the time dependent release of solubilized Nile Red in FaSSGF buffer pH 1.2 from MENIII@ZIF-8 is depicted. The squares show the normalized concentration (mean ± SD, n=10) of solubilized Nile Red referred to the value before ZIF-8 synthesis as 100%. It can be seen, that the amount of solubilized fluorescent dye Nile Red increases over time. Already at 0.10 h a significant amount of Nile Red is solubilized demonstrating a fast response of the loaded ZIF-8 to the change of pH-value as a chemical stimulus. This also demonstrated the formation of a microemulsion and the formation of a SMEDDS (indicated by the formation of the microemulsion without applying any measures for preparing an emulsion) both in response to the change of the pH-value using the loaded ZIF-8. Furthermore, the increase of the solubility of the poorly water-soluble fluorescent dye Nile Red was demonstrated. After 1 h an amount of about 50% of Nile Red was already solubilized and the amount further increased over time which lead to a solubilized Nile Red amount of more than 55% after 6 h. After 24 h the amount of the solubilized Nile Red was about 60%.

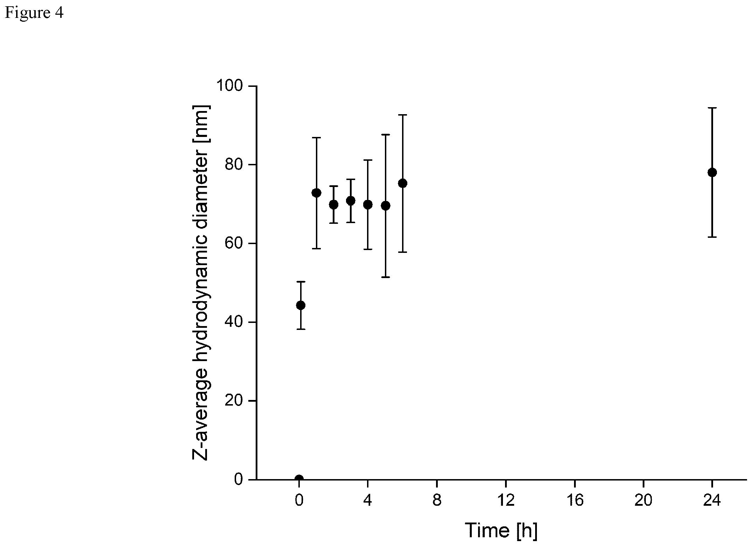

In Figure 4 the results of the dynamic light scattering measurements of the aliquots are shown. Figure 4 shows the size of colloids in FaSSGF buffer pH 1.2 from MENIII@ZIF-8 over time. The circles show the Z-average hydrodynamic diameter in nm of colloids (mean ± SD, n=10) indicating the formation of a microemulsion at pH 1.2. As can be seen from Figure 4 at 0.10 h colloids having a Z-average hydrodynamic diameter were formed, which indicates that in response to the change of the pH-value which is a chemical stimulus the formation of a microemulsion occurred quickly. These results correspond to the results of the fluorescence intensity measurement. Moreover, already after 1 h a microemulsion having a relatively stable droplet size was formed. After 24 h the Z-average hydrodynamic diameter was about 80 nm. These results, indicate that a microemulsion was formed in response a change in pH-value which is a chemical stimulus, without applying a measure for preparing a microemulsion.

Therefore, the results displayed in Figures 3 and 4 indicate that from the composition containing the MENIII loaded ZIF-8 which is a solid dosage form of a SMEDDS according to the present invention, in response to a change in pH-value which is a chemical stimulus, a liquid self-microemulsifying system was generated, which lead to the formation of a microemulsion and consequently to the increase of the solubility of the fluorescent dye Nile Red in the water-based buffer FaSSGF.

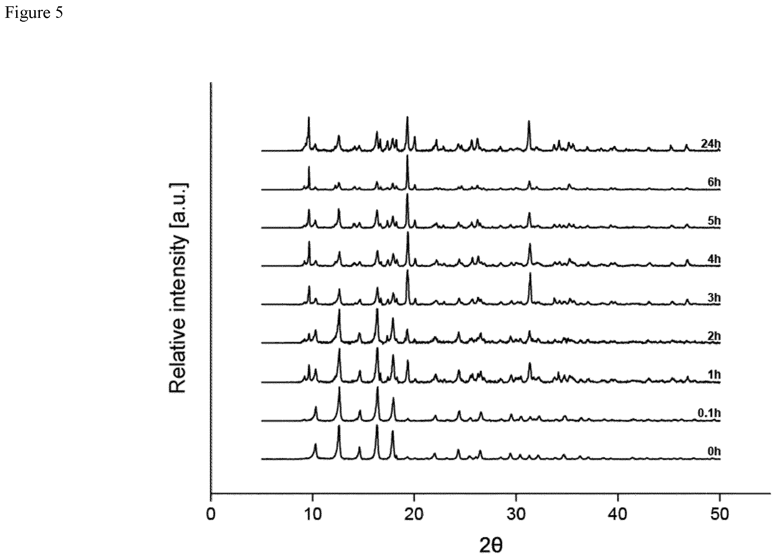

From the aliquots obtained in Example 3 the contained solids were isolated. In Figure 5 the results of the XRPD measurements of these solids contained in the aliquots obtained from Example 3 are displayed. ZIF-8 is biodegradable under acidic conditions and therefore suitable as pH-responsive carrier material for a solid dosage form of a SMEDDS, as can be seen from Figure 5 displaying the structural changes of MENIII loaded ZIF-8 over time at a pH-value of 1.2.

### Example 4: Light responsive: MENIII@UiO-AZB

- UiO-AZB was synthesized as previously reported by Roth Stefaniak, K.; Epley, C. C.; Novak, J. J.; McAndrew, M. L.; Cornell, H. D.; Zhu, J.; McDaniel, D. K.; Davis, J. L.; Allen, I. C.; Morris, A. J.; Grove, T. Z., Photo-triggered release of 5-fluorouracil from a MOF drug delivery vehicle. Chem Commun (Camb) 2018, 54 (55), 7617-7620. Yield: 1.2 g with 83% UiO-AZB, 6.8% lipids and 10.2 % emulsifiers.
- UiO-AZB was loaded with 0.4% SMEDDS MENIII by impregnation for 24h.
- Loaded samples were immersed in FaSSIF V1 buffer pH 6.5 at 37°C and irradiated with 340 nm light for 24h.
- Aliquots were taken at 0.1, 1, 2, 3, 4, 5 ,6 and 24h and microemulsion desorption was determined by fluorescence intensity measurement and dynamic light scattering.

The components adsorbed to the MOF have been desorbed from the MOF upon irradiation with the light. This led to photoisomerization of AZB linker and breakdown of the MOF and released adsorbed and encapsulated components

### Example 5: Temperature responsive: MENIII@ZJU-64

- ZJU-64 was synthezied as previously reported by Lin, W.; Hu, Q.; Yu, J.; Jiang, K.; Yang, Y.; Xiang, S.; Cui, Y.; Yang, Y.; Wang, Z.; Qian, G., Low Cytotoxic Metal-Organic Frameworks as Temperature-Responsive Drug Carriers. Chempluschem 2016, 81 (8), 804-810. Yield: 1.3 g consisting of 87% ZJU-64, 5.2% lipds and 7.8% emulsifiers.
- Adenine and [1,1':4',1"-terphenyl]-4,4"dicarboxylicacid were dissolved in DMF.
- An aequos Zn(NO₃)₂·6H₂O solution containing HNO₃ and 1% SMEDDS MENIII was added.
- The mixture was heated at 130°C for 24h.
- After cooling, the product was obtained by filtration.
- Samples were immersed in FaSSIF V1 buffer pH 6.5 at 37°C or 60°C for 24h.
- Aliquots were taken at 0.1, 1, 2, 3, 4, 5, 6 and 24h and microemulsion desorption was determined by fluorescence intensity measurement and dynamic light scattering.

The components adsorbed to the MOF have been desorbed from the MOF upon increasing the temperature to 60°C. ZJU-64 degraded at high temperature releasing attached components.

### Example 6: Pressure responsive: MENIII@ZJU-800

- ZJU-800 was synthesized as previously reported by Jiang, K.; Zhang, L.; Hu, Q.; Zhao, D.; Xia, T.; Lin, W.; Yang, Y.; Cui, Y.; Yang, Y.; Qian, G., Pressure controlled drug release in a Zr-cluster-based MOF. J Mater Chem B 2016, 4 (39), 6398-6401. Yield: 1.16 g with 83% ZJU-800, 6.8% lipids and 10.2% emulsifiers.
- ZJU-800 was loaded with 0.4% SMEDDS MENIII by suspending both in aqueous solution at room temperature for 24h.
- After centrifugation and drying, samples were immersed in FaSSIF V1 buffer pH 6.5 at 37°C for 24h with a pressure of 0, 30 or 60 MPa for instance.
- Aliquots were taken at 0.1, 1, 2, 3, 4, 5, 6 and 24h and microemulsion desorption was determined by fluorescence intensity measurement and dynamic light scattering.

The components adsorbed to the MOF have been desorbed from the MOF upon increasing the pressure to 0, 10 or 30 MPa. Changes in pressure led to "burst release" of adsorbed and encapsulated components from ZJU-800. The lower the pressure the faster the release.

### Example 7: Redox responsive: MENIII@MOF-Zr(DTBA)

- MOF-Zr(DTBA) was synthesized as previously reported by Lei, B.; Wang, M.; Jiang, Z.; Qi, W.; Su, R.; He, Z., Constructing Redox-Responsive Metal-Organic Framework Nanocarriers for Anticancer Drug Delivery, ACS Appl Mater Interfaces 2018, 10 (19), 16698-16706. Yield: 520 mg with 96% MOF-Zr(DTBA), 1.6% lipids and 2.4% emulsifiers.
- MOF-Zr(DTBA) was loaded with 0.4% SMEDDS MENIII by suspending both in aequos solution at room temperature for 24h.
- After centrifugation and drying, samples were immersed in FaSSIF V1 buffer pH 6.5 at 37°C containing DTT/GSH under continuous stirring for 24h.
- Aliquots were taken at 0.1, 1, 2, 3, 4, 5, 6 and 24h and microemulsion desorption was determined by fluorescence intensity measurement and dynamic light scattering.

The components adsorbed to the MOF have been desorbed from the MOF upon initiating a redox-process in the presence of DTT/GSH. Disulfide bond in DTBA is GSH sensitive which led to degradation of the MOF and release of attached and encapsulated components.

## Claims

1. A composition comprising:
a) a metal-organic framework;
b) one or more emulsifier(s); and
c) one or more lipid(s).

2. The composition according to claim 1, wherein the one or more emulsifier(s) and the one or more lipid(s) are adsorbed to the metal-organic framework, and wherein the one or more emulsifier(s) and the one or more lipid(s) can be desorbed from the metal-organic framework in response to a physical and/or chemical stimulus.

3. The composition according to claim 2, wherein the physical stimulus is selected from the group consisting of: temperature, pressure, light, sonic waves and mechanical stress.

4. The composition according to claim 2, wherein the chemical stimulus is selected from the group consisting of: a change in pH-value, initiating redox-processes and a presence of an enzyme.

5. The composition according to claims 1 to 4, wherein the composition further comprises:
d) one or more pharmaceutically active ingredient(s).

6. The composition according to claims 1 to 5, wherein the metal-organic framework is a zeolitic imidazolate framework.

7. The composition according to claim 6, wherein the metal-organic framework is the zeolitic imidazolate framework ZIF-8.

8. The composition according to anyone of claims 1 to 7, wherein the composition further comprises:
e) one or more co-emulsifier(s).

9. The composition according to anyone of claims 1 to 8, wherein the one or more emulsifier(s) are non-ionic emulsifiers.

10. A method for preparing a microemulsion in response to a physical- and/or chemical stimulus comprising the following steps:
i) providing a composition comprising:
a) a metal-organic framework;
b) one or more emulsifier(s);
c) one or more lipid(s), and
d) one or more solvent(s);
ii) applying a physical and/or chemical stimulus to the composition.

11. The method according to claim 10, wherein in step ii) a chemical stimulus is applied to the composition.

12. Use of a composition comprising:
a) a metal-organic framework;
b) one or more emulsifier(s); and
c) one or more lipid(s);
for preparing a microemulsion in response to a physical- and/or chemical stimulus.

13. A method for increasing the solubility of a pharmaceutically active ingredient in a solvent and/or for improving the bioavailability of a pharmaceutically active ingredient and/or for preparing a self-microemulsifying drug delivery system comprising the following steps:
iii) providing a composition comprising:
a) a metal-organic framework;
b) one or more emulsifier(s);
c) one or more lipid(s);
d) one or more pharmaceutically active ingredient(s); and
e) one or more solvent(s);
iv) applying a physical and/or chemical stimulus to the composition.

14. The method according to claim 13, wherein in step iv) a chemical stimulus is applied to the composition.

15. Use of a composition comprising:
a) a metal-organic framework;
b) one or more emulsifier(s); and
c) one or more lipid(s);
for increasing the solubility of a pharmaceutically active ingredient in a solvent and/or for improving the bioavailability of a pharmaceutically active ingredient and/or for preparing a self-microemulsifying drug delivery system.
